# EUROPEAN PATENT APPLICATION

(11) **EP 2 570 950 A1**
(43) Date of publication of application: **20.03.2013**
(21) Application number: 09848905.7
(22) Date of filing: 07.09.2009
(51) Int. Cl.: G06F 19/00, G06Q 50/00

(54) **SYSTEM FOR THE INTEGRAL CONTROL AND MANAGEMENT OF THE MEDICAL RECORDS OF PATIENTS IN HEALTH CENTRES, HOSPITALS, OUTPATIENT CENTRES AND THE GENERAL HEALTHCARE SYSTEM**

(71) Applicant: Personalized EMR, LLC, d/b/a Trabajando como MediBANK International, Dallas 75201 (US)
(72) Inventor: VIÑALS LARRUGA, Javier, E-28023 Madrid (ES)
(74) Representative: Baños Treceño, Valentín
(86) International application number: PCT/ES2009/000441
(87) International publication number: WO 2011/027006

(57) **Abstract**

The invention relates to a system for storing and managing the complete medical records of patients, which essentially comprises a system that allows the common user to access all of his/her medical information, as well as an operation that can be used to monitor and manage the continuity of assisted care system, with the following order of action: a periodic review of all the protocols or templates activated during a given time period, the entry into the monitoring system of the periodicity recommended by the doctor and automatic review of compliance by the patient.

The following steps are performed upon detection of the non-receipt of data namely:-A call center contacts the user; the doctor is informed and the protocol is cancelled; the user is offered support and the necessary assistance is provided; in addition, the doctor is sent a report, and the patient is reclassified to ensure that he/she is more closely monitored in the future.

## Description

### PURPOSE OF THE INVENTION

As indicated by the title, the invention consists of a computer system for the full control and management of medical records and the medical assistance provided to patients in healthcare centers, hospitals, outpatient centers, and in particular throughout the whole national and international healthcare network, where physicians, specialists and other professionals may interact in order to update, maintain or correct the medical records or the patient's assistance requirements. The system also allows the patient's interaction by including all the necessary tools that assure a good performance of the users of the program, which throughout the memory will be denominated "the system".

### BACKGROUND OF THE INVENTION

There are local computer programs that are basic tools located in each center which are limited to each specialty that allow to know the medical record of a patient that belongs to that center through a single registry where the chronological development of the assistance provided to the patient in that center may be verified by a particular physician, a nurse, a family doctor or any other specialist.

### DESCRIPTION OF THE INVENTION

The system may be broken down in the following fields:
1. A system that includes the following functionalities:
   The introduction of data through the WEB
   1.2. The introduction of data by Safe Fax (Esc. Func)
   1.3. The introduction of data via CONNECTIVITY (Id)
2. A system that includes the following functionalities:
   2.0 for Owner of the medical records:
   2.1. for extraction of information through the web - full report
   2.2. for extraction of the report through the web - specific extraction
   2.3. for extraction of information through the web -report's descriptive interface
3. System that includes the following functionalities:
   3.1. Health Assistance Diary - screen of summary
   3.2. Health Assistance Diary - main screen
4. System that includes the following functionalities:
   4.1. for General Prevention
   4.2. for Prevention by profile
5. System that includes the following functionalities:
   5.1. for extraction of Information from EMERGENCIES using a PIN
   5.2. for CONTINUITY OF ASSISTED CARE (C.A.C.) (1)
   5.3. for CONTINUITY OF ASSISTED CARE (C.A.C.) (2)
   5.4. for CONTINUITY OF ASSISTED CARE (C.A.C.) (3)
   5.5. for CONTINUITY OF ASSISTED CARE (C.M.A.) (4)
6. System that comprises the following functionalities:
   6.1. INTEGRATION OF SYSTEMS
   6.2. for ADJUSTMENT, EMBEDDING AND MIMECRY
   6.3. for PROFESSIONAL
   6.4. for PROFESSIONAL (1)
   6.5. for PROFESSIONAL (2)
   6.6. for PROFESSIONAL (3)
   6.7. for the web of C.M.A. to recommend protocols
   6.8. for the hosting and connectivity web
   6.9. for Technical Maintenance and Technological Support
   6.10 for Monitoring and Managing the correct connectivity of data coming from health information systems
7. System that comprises the following functionalities:
   7.1. telephone recommendation depending on patient's medical history
   7.2. for Proactive Recovery and Monitoring of Medical Reports Flows
   7.3. for Classification of Types of Reports and ICD Codes
   7.4. Call Center for Medical Support in cases of incidents with the introduction or removal of information
   7.5. for Monitoring and managing the CAC and resolving problems in the flow of information between doctors and patients.

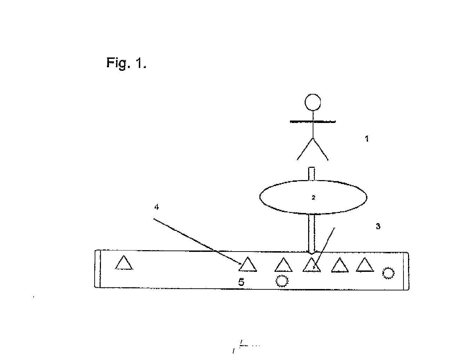

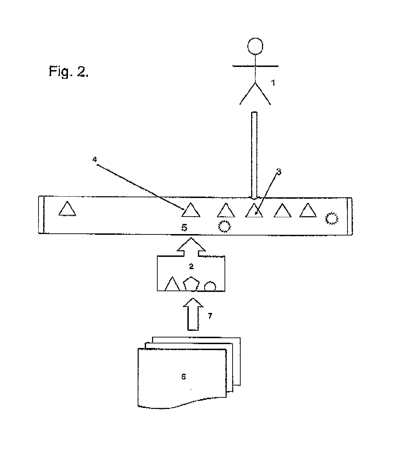

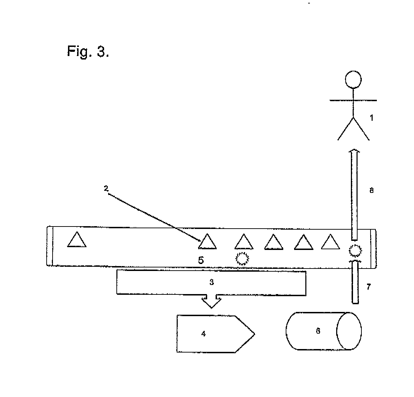

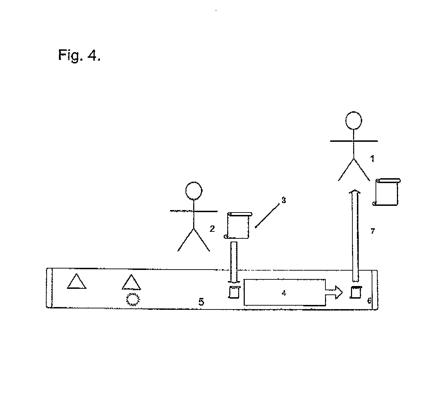

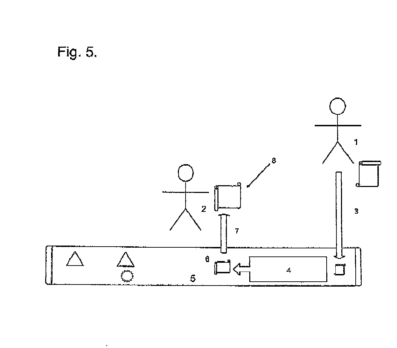

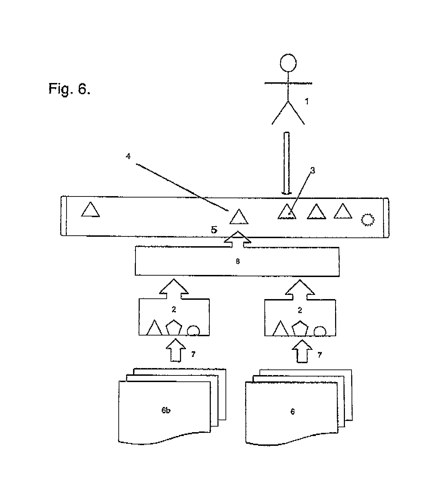

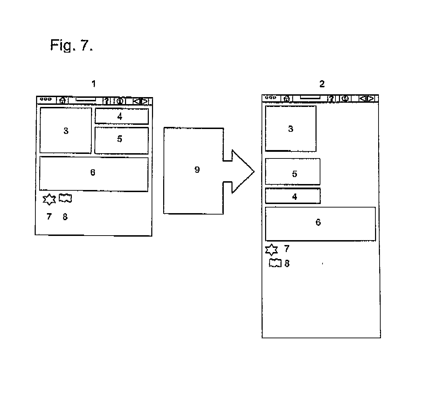

1.1. A functionality consisting of the introduction of data through the web that has a screen which allows the patient to access a form and choose a file (scanned from the Clinical Report to be registered) to be added to his/her database. The form of the file admitted comprises:
   - JPEG
   - GIF
   - TIFF
   - BMP
   - PDF
   Additionally to same user is allowed to introduce certain information associated to the documents that is unloaded in the server:
   - Date of report
   - Author doctor
   - Specialty
   - Center of origin
   - Type of report
   All this information is classified in the database of the system as INFORMATION NOT VERIFIED from the medical point of view.
   Later on, as part of another procedure, the system offers the possibility of a medical review and a subsequent classification (ICD) by the part of a practicing doctor.
1.2. A functionality consisting of the introduction of data by Safe Fax (see functional scheme of Fig. 1) by means of which the user is provided with a single code of identification, and a single and private fax number made up by 9 initial digits plus the individual code assigned. By dialing this full code, the document sent by fax will be automatically deposited in the personal data base of medical reports of the user.
1.3. A functionality consisting of the introduction of data via CONNECTIVITY (see functional scheme of Fig 2 Page) through connection links or direct connectivity with the 10 system, by depositing a copy of the report directly in the central data base of the system. To do so, a previous relation will be set up between the identifier of the original system of information (usually the Hospital Information System) and the single identifier of the user of the system. Likewise, the fields that will be part of the set of information that will be available to the user through the system will be previously defined and transferred, with his/her consent, from one system to the other.
2.0. A functionality consisting of the verification of the Owner of the medical report (see functional scheme Fig 3, Page...) that allows the user to obtain a quick and accurate image of his/her medical profile at first glance. The purpose of the diagram is to represent in one axle the time scale while the other axle shows the columns that depict the several medical areas that divide the medical specialties (cardiology, traumatology, neurology, etc.).
   The peculiarity that allows this functionality to provide so much important information in such a small space does not only dwell on the organization of the space but also on the icons and symbols representing each report. These icons and symbols include the following elements:
   - Acronym of 3 letters: that defines the type of report (LAB, RAX= Radiology X Rays, RMN = Magnetic Resonance Imaging (MRI)
   - Exact date
   Additionally the diagram gathers in a clearly visible manner the reports that are part of a single clinical episode. For example, in case of urgent surgery, a clinic episode would be made up by the following individual reports:
   - Urgency report
   - Lab report
   - Report concerning the preanesthetic preparation
   - Surgical Report
   - Continuation of hospitalization report
   - Discharge of patient report
2.1. A functionality consisting of extracting the information from the web - full report- made up of two options:
   A: Extraction of full report
   B: Specific extraction and search of reports
      The user has an interface that allows him/her to choose:
      - Range of dates of insertion for search (initiation/final)
      - Range of dates for creation of search (initiation/final)
      - Specialties
         - Center of origin
         - Type of report
         - Doctor / Report's inspector
         - CIE Code
   Likewise the interface allows the user to sort out the result of the search in accordance with the privacy level (visible or non visible) assigned to each report by the user. A classified listing is obtained that allows the user to interact choosing with the mouse the report to be consulted. Once selected, the user may access a screen that allows him/her to see the basic areas of the report:
   - Codified information
   - Text of report
   - Images or documents attached
2.2. A functionality consisting of the Extraction of Information through the web - specific extraction- that comprises two options:
   A: Extraction of the full report
   B: Specific extraction and search of report
      The user has an interface that allows him/her to choose:
      - Range of dates of insertion for search (initiation/final)
      - Range of dates of creation for search (initiation /final)
      - Specialties
      - Center of origin
      - Type of report
      - Doctor/Inspector of report
      - CIE Code
      Likewise, the interface allows the user to sort out the results of the search in accordance with the level of privacy (visible or non visible) the user assigns to each report. A classified listing is obtained that allows the user to interact choosing the report to be consulted by using the mouse. Once selected, the user accesses a screen that allows him to see the areas of the report:
      - Codified information
      - Text of report
      - Images or documents attached
2.3. A functionality consisting of the extraction of the information from the web - descriptive interface of the report- that includes two options:
   A: Extraction of the full report
   B: Specific extraction and search of reports
   The user has an interface that allows him/her to choose:
   - Range of dates of insertion for the search (initiation/final)
   - Range of dates of creation for search (initiation/final)
   - Specialties
   - Center of origin
   - Type ofreport
   - Doctor / Inspector of Report
   - CIE Code
   Likewise the interface allows the user to sort out the result of the search in accordance with the level of privacy (visible or non visible) that user assigns to each report.
   A classified listing is obtained that allows the user to interact, choosing with the mouse the report to be consulted. Once selected, the user accesses a screen that allows him to see the basic areas of the report:
   - Codified information
   - Text of report
   - Images or documents attached
3.1. A functionality consisting of a verification of the Health Diary - screen of summary- that offers a double option:
   - Screen of summary that allows the user to have access, from the central page of collection of global information over his/her data, to:
   - A system of summaries of personal health that indicates the general condition and quality of life of the user offering a detailed explanation of the data provided by him/her throughout the time. The information is broken down in specific windows for:
   - Control of weight
   - Control of healthy life habits
   - Control of diet
   - Control of physical exercise
   - Personal agenda that allows the user to mark appointments and reminders of dates. These appointments may be associated to alarms that are sent to the user by e-mail or SMS messages to the cell phone.
   - Main screen
3.2. A functionality consisting of a verification of the Health Diary - main screen that includes a double system:
   - Summary screen that allows the user, from the central page of collection of global information over his/her data, to have access to:
   - Summary of personal health that indicates the general condition and the quality of life of the user offering a detailed explanation of the data provided by such user throughout the time. At the same time specific windows are offered for
   - Control of weight
   - Control of healthy life habits
   - Control of diet
   - Control of physical exercise
   - Personal agenda. Allows the user to write down appointments and reminders on specific dates. These appointments may be associated to alarms that are sent to the user by e-mail or SMS messages to the cell phone.
   - Main screen. Allows to access to more precise information and also to follow up charts of each one of the basic parameters. Besides, it offers a form where the user may update when considered appropriate the basic data that concerns his/her personal health.
4.1. General Prevention Functionality which consists of several processes that provide the user with information about any prevention protocol published either by the World Health Organization of the Ministry of Health or Groups of Consensus. The information is not sorted out and is received by the user exactly as published by the source of origin.
   The system makes an update and constantly renews the information through seasonal modifications.
   In general terms, these are protocols that have an impact on the population as a whole which means that their nature should be considered rather common. In this way the users of the system do not have to pay much attention to the different origins where these campaigns are published as they only access from their personal page of medical data with the certainty that they will never miss any relevant information.
   Two areas are offered:
   - Window of summary on the personalized page of the user
   - Window offering detailed explanations (higher or lower depending on the content of the protocol in question)
4.2. A functionality of Prevention per profile consisting of processes that provide the user with information about any protocol of prevention published either by the World Health Organization, the Ministry of Health of Groups of Consensus.
   In this case the information is sorted out and crossed with the patient's data stored in the system, basically with data corresponding to:
   - Age
   - Sex
   - Weight
   - Smoking habits
   - Pharmacological habits
   - Stress
   - Diet habits
   - Personal background
   - Family background
   An additional prevention may be made with the above premises with a high degree of sophistication and specialty in such a way that the user will not need to pay a visit to the doctor's office or clinic to know that he/she should be included in specific groups of risk.
   The functionality at the same time includes:
   - A summary screen that shows the results of the crossing of information with the data base of all existing prevention protocols for the several groups of risk
   - A specific screen that offers explanations about each protocol and particularly, the possibility of making a personal and self controlled follow-up.
5.1. A functionality that consists of the Extraction of Information from EMERGENCIES using a PIN. All users, when registering in the system besides receiving the name and a password receive a special number called EMERGENCY PIN. By using this PIN (either through the web or the telephone), the user is immediately provided with the full medical report stored in the system which is sent to the appropriate address previously provided (by electronic mail, fax or direct printing through the web).
   Once entered, the system disables the EMERGENCY PIN and generates a new one that is sent to the user. The purpose of this procedure is to disable and avoid the possibility of repeated entries to the system through non authorized means.
5.2. A functionality that consists of the verification of the CONTINUITY OF ASSISTED CARE (CAC) 1 where the process owner of the system has two main components:
   A) A functionality of individualized criteria through the web (for doctors), which is a safe way accessible through the web that allows the doctor to select a specific patient and recommend a simple personalized protocol. Besides establishing the guidelines to be controlled, it may also establish the periodicity as well as the remittance and picking up procedures. The fields contemplated within this system are:
      A) Name of protocol (protocols of common or standard use may be kept):
      B) Name of parameter
      C) Maximum value to be assessed
      D) Minimum value to be assessed
      E) Follow up modality (how the information is received)
      F) Common follow up procedure
   B) System that allows a collective as well as a detailed review of the patient's data. The doctors that are users of the system may access a web that allows them:
      A) To review a global chart of the health condition of the patient, its progression and any remark about the patients under treatment.
      B) To review in detail the partial progression of a specific patient
   The users simply receive a message (SMS), an e-anail or a telephone call including the value of each field involved.
   The doctor will have the possibility of calling the patient to consultation in case of finding any deviation in the monitoring graphics. The functionality is addressed to avoiding the permanent attendance of the patient to the doctor's office for check-ups and also to avoid admissions in urgencies for lacks of follow-up.
5.3. A functionality that consists of the verification of the CONTINUITY OF ASSISTED CARE (CAC) 2 where the process owner of the system has two main components:
   A) A functionality of individualized criteria through the web (for doctors), which is a safe way accessible through the web that allows the doctor to select a specific patient and recommend a simple personalized protocol. Besides establishing the guidelines to be controlled, it may also establish the periodicity as well as the remittance and picking up systems. The fields contemplated within this system are:
      A) Name of protocol (protocols of common or standard use may be kept):
      B) Name of parameter
      C) Maximum value to be assessed
      D) Minimum value to be assessed
      E) Follow up modality (how the information is received)
      F) Common follow up procedure
      B) System that allows a collective as well as a detailed review of the patient's data. The doctors that are users of the system may access a web that allows them:
      A) To review a global chart of the health condition of the patient, its progression and any remark about the treatment.
   B) To review in detail the partial progression of a specific patient
   The users simply receive a message (SMS), an e-mail or a telephone call including the value of each field involved.
   The doctor will have the possibility of calling the patient to consultation in case of finding any deviation in the monitoring graphics. The functionality is addressed to avoiding the permanent attendance of the patient to the doctor's office for check-ups and also to avoiding admissions in urgencies for lack of follow-up.
5.4. Functionality to verify the CONTINUITY OF ASSISTED CARE (CAC) (3) where the process owner of the system has two main components:
   A) A functionality of individualized criteria through the web (for doctors), which is a safe way accessible through the web that allows the doctor to select a specific patient to recommend a simple personalized protocol. Besides establishing the guidelines to be controlled, it may also establish the periodicity as well as the remittance and the picking up systems. The fields contemplated within this system are:
      A) Name of protocol (protocols of common or standard use may be kept):
      B) Name of parameter
      C) Maximum value to be assessed
      D) Minimum value to be assessed
      E) Follow up modality (how the information is received)
      F) Common follow up procedure
      B) System that allows a collective as well as a detailed review of the patient's data. The doctors that are users of the system may access a web that allows them:
      A) To review a global chart of the health condition of the patient, its evolution and any remark about the treatment.
   B) To review in detail the partial progression of a specific patient
   The users receive a SMS, an e-mail or a telephone call including the value of each field recommended.
   The doctor will have the possibility of calling the patient to consultation in case of any deviation in the monitoring graphics. The functionality is addressed to avoiding the permanent attendance of the patient to the doctor's office for check-ups and also to avoiding admissions in urgencies for lack of follow-up.
5.5. Functionality to verify the CONTINUITY OF ASSISTED CARE (CAC) (4)) A functionality of individualized criteria through the web (for doctors), which is a safe way accessible through the web that allows the doctor to select a specific patient and recommend a simple personalized protocol. Besides establishing the guidelines to be controlled, it may also establish the periodicity as well as the remittance and picking up systems. The fields contemplated within this system are:
   A) Name of protocol (protocols of common or standard use may be kept):
   B) Name of parameter
   C) Maximum value to be assessed
   D) Minimum value to be assessed
   E) Follow up modality (how the information is received)
   F) Common follow up procedure
   B) System that allows a collective as well as a detailed review of the patient's data. The doctors that are users of the system may access a web that allows them:
   A) To review a global chart of the health condition of the patient, its evolution as well as any remark about the treatment.
   B) To review in detail the partial progression of a specific patient
   The users simply receive a message (SMS), an e-mail or a telephone call including the value of each field involved.
   The doctor will have the possibility of calling the patient to consultation in case of detecting any deviation in the monitoring graphics. The functionality is addressed to avoiding the permanent attendance of the patient to the doctor's office for check-ups and also to avoiding admissions in urgencies for lack of follow-up.
6.1. Functionality for the INTEGRATION OF THE SYSTEMS (see functional scheme Fig. 4 Page) which contains the elements that allow the incorporation of its data into any Hospital Information System, First Aid Systems or Management Systems of Medical Reports in doctor's offices or clinics.
   The product offers an exact definition of the fields to be imported:
   - Name of report
   - Identification of patient
   - Name of patient
   - Basic data concerning the patient's affiliation
   - Text of report to be delivered
   - Set of files added (images etc.)
   - ICD code
   - Internal code of classification and indexing
   - Specialty
   - Date
   - Type
   - Opening or closing index of the episode
   Additionally, basic Application Programming Interfaces have been developed that allow other processes to communicate with the system in a smooth manner. The system includes a full set for the communication of the fields described above as well as a general definition for future developments.
6.2. A functionality for ADJUSTMENT, EMBEDDING AND MIMECRY, which has been developed in blocks of exchangeable codes and XML templates that allow an easy integration in the web portals of other institutions.
   Besides these templates allow the quick modification of:
   - Color schemes
   - Subject schemes
   - Size of letters and types of headings
   - Schemes of relational placement
   The above allows that the functionality may be used in a different area by providing the brand image of a different company but using the operation "motor" and the data base of the system, all of it in a very transparent manner for the final user.
6.3. Functionality for PROFESSIONALS. Once in the application we will find a window which configuration will depend on the profile of our user. This functionality will be explained in the user's manual through an intricate window, the one corresponding to the supervisor's profile, to subsequently show at the end of the document the several profiles of the users that are available and the limitations of each one of them. Therefore, hereunder we will explain each one of the tables or charts included in the application as well as the menus and the information provided.
   2.1. General information
   Within the application we will find the following general information:
   - Information about the name of the application followed by the organization we are connected to (marked as 1 within the above chart)
   - Information about the type of profile our user has, which will condition the operations we may carry out and the data we will be able to visualize (marked as 2 in the chart)
2.2. Menus of the application.
   Within the application there will be three menus which are described as follows:
   - Menu file
   Within this menu we have the options, Save, Change General Data, Change of Password, Medications Data Base and Exit.
   The Save submenu will allow us to keep at any times all the changes made in the data included in the application (of the patient, reports, etc.)
6.4. PROFFESIONAL functionality (1) where the submenu Change of General Data will allow us to modify the general data of our user, i.e. of the user that is connected to the application.
   Within this window modifications can be made in the first and family names, the number of association and the electronic mail.
   The submenu Change of Password will allow us to modify our password of access to the application. To do so, we have to select the option which will take us to a window where we will be requested the password of access the current application as well as the new password. For the change to become effective we have to press OK. If we do not want to change our password of access we must press "Cancel".
   The Medications Data Base submenu will take us to the page of publications made by the Ministry of Health and Consumption from where we can have access to all medications and their active ingredients.
   The Exit submenu is used to get out of the application. In case of having made any change in the information stored or in case of adding new data the system will ask whether or not the changes should be kept, if we want to cancel the operation or close the application
6.5. A PROFESSIONAL (2) functionality which uses a:
   - Menu Tools
      Within this menu we have the menus Preferences and Edit Health Institution.
      The Preference submenu allows us to access another screen where we may arrange the characteristics of the application. Specifically:
   - The location of the medications database in our system, or the access to a free version of it in case of not having one:
      To keep the changes made first we must press "Apply" and then "OK".
   - Configuration of Data to be sent out: allows us to organize the data, the parameters of the server and the account from where the clinic will send the mails to its patients: Besides this window will indicate, through the appropriate marks, if the clinic will be sending the passwords of access to the patient by electronic mail or by SMS through the application created to such purpose and if the patient must be advised by e-mail or SMS when a report is created for him/her by the system.
   The same as in the previous case, in order to keep the changes made we must first press "Apply" and then "OK".
   Language: allows choosing the language in which the texts and messages will be shown. Currently the options offered are Spanish, English and Portuguese.
6.6. PROFESSIONAL functionality (3) in which, the same as the previous ones, in order to keep the changes made we need first to press "Apply" and then "OK"
   - Network Connections: allows to modify the configuration of access to the Internet in case the clinic has a Proxy:
   - The "Core" and "Core Global" options are only accessible to the application administrator, so if we choose them we will obtain the following information:
   The submenu Edit Health Institution allows us to go to another screen where we can modify the data of our clinic. We can change the name, the description or add or delete insurance companies our clinic works with.
   To carry out this operation we must press "Add Insurer". A list of all existing insurance companies will appear and the insurers that already work with our clinic will be specifically marked. We may select or delete insurers from this list:
   After choosing the insurer wanted we must press "OK" and the companies chosen will be immediately associated to our clinic.
   - Menu Help
   If we select this menu we may see the technical information of the application.
6.7. A functionality of the CAC web to organize protocols through the web in a personal and individualized manner (for doctors)
   By using a simple protocol, which is accessible in a safe manner through the web, the doctor is allowed to choose a specific patient and start a simple personalized protocol. Besides establishing the parameters to be controlled the doctor may also establish the periodicity and the remittance and picking-up methods.
   The fields contemplated within the system are:
   A) Name of protocol (protocols of usual or standard use may be kept)
   B) Name of parameter
   C) Maximum value to be assessed
   D) Minimum value to be assessed
   E) Follow-up method (how the information is received)
   F) Common follow- up option.
6.8. Web functionality for Hosting and Connectivity (see Fig 4 page) where the specific service called Dedicated Hosting is so called because specifically uses a group of servers which are available exclusively to the users of the system. The process contains its own operating system along with the required applications to maintain the whole operation. Besides it provides an own accommodation, all the elements concerning the operation and the physical maintenance including an appropriate and fitted-out space, a climate system, the required electric feeding, all the installation works, the connections and the start up as well as the permanent maintenance for the hardware. This concept also includes all the requirements concerning the support and maintenance of the operating system and the applications installed, and in particular a continued updating to avoid safety issues. Other services included are: CONNECTIVITY or connection to the Internet, private connections (VPN) to facilitate the exploitation of the client's data, protection with firewalls and detection of intrusions, safety copies, personalized monitoring of all components and applications, reports and statistics of use, events, consumption, accounting of transactions, etc. The above services are available on permanent basis and also backed up by a 24x7 center of operations. Certain clients are offered partial access to service data and statistics through a control panel in the web.
6.9. A functionality of Technical Maintenance and Technological Support (see Fig 5 page) where the specific service of Technical Support includes all those functions dedicated to the permanent operation of the system. This functionality includes a Technical Call Center or center for the attention of incoming and outgoing calls; it is a tool of communication and relation with the users through the telephone as a way of basic communication handled by administration agents with an appropriate degree of qualifications. The activity of the technical experts is addressed to maintaining a set of the necessary and required human, physical and technological resources, all of them based on work methodologies and specific and appropriated processes to meet the service requirements of each user in order to maintain and sustain their trust and preserve their loyalty with the system.
6.10 Functionality to Monitor and Manage the correct connectivity of the data coming from the information systems of health centers (see Fig 6 page) where the specific service to Monitor and Manage the correct CONNECTIVITY of data coming from external systems is a key element in the support of the basic function which is the integration of information from several sources with total transparency for the user.
   The system provides a "monitoring channel" or data flow for verification with each supplier of medical data incorporated, this is, with any health system which clinical data has been incorporated to the system.
   Additionally an "autonomous monitoring channel" is provided for the information coming from the PROFESSIONAL system.
   The data verified on permanent basis is:
   - Status of the operation "open" to the channel
   - No intrusion filter
   - Monitoring of the expected correct volume of data within a specific period of time.
   - Random monitoring of blocks of patients in anonymous form An analysis report is issued on regular basis with the magnitudes picked up during the permanent verification and the process that handles the operation of the connection channels.
   If any anomaly is detected in the parameters expected for each channel, the system activates an alarm destined to handle the incident internally within the technical maintenance service and also through a communication with the source of origin of the data from the channel affected.
7.1. A functionality for the Patient's Medical History (see Fig. 7 page). A process that allows in an optimal manner to deploy and start up the user's Pi-IR. Through a specific protocols based on the Patient's Medical History, a qualified doctor will call the user to set the basis of his/her history in the storage mode. Previously, the administration mode has been in contact with the user, who has previously accepted and given his/her consent to the procedure.
   The administrator specifies the most convenient time for the patient to be called by one of the doctors from the "doctor's pools" stored in the system.
   One of these doctors will conduct the full interview, introduce the data in the personal account of the user, give the appropriate instructions to this user about the future use of the system to finally establish the best protocol to be applied on permanent bases (See Annex 19).
   Structure of the system's interview:
   - Salutation and initiation: Duration from 5 to 20 seconds
   - Communication of Basic Data (LOPD) and establishing a degree of empathy: Duration: from 30 to 60 seconds
   - Psychological Analysis and psycho gram. Duration: from 1 to 2 minutes
   - Reason of the interview: initial construction of the medical profile for the system. Duration. 20 seconds.
   - Exploration of brief profile for the system. Special capture of clinical data about initiation, intensity, duration, frequency, association with events or situations. Duration: from 3 to 6 minutes.
   - Direct history of family background. Duration: from 1 to 2 minutes
   - Direct history of personal background. Durations: from 1 to 2 minutes.
   First Childhood (0 to 6 years of age)
   Second Childhood (6 to 12 years of age)
   Adolescence (12 until reaching a socio-economic independence)
   Young adulthood (up to 40 years of age)
   Mature adulthood (40 to 65-70 years of age)
   Senility (more than 70 years of age)
   Explanation of conclusions to patient: Duration. 30 seconds. Goodbye and salutation. Duration. 15 seconds.
   Minimum total duration. 7.5 minutes
   Maximum total duration. 14,5 minutes
   Average duration. 11 minutes
   Durations are only an indication, are not mandatory and may change from case to case.
7.2. Functionality for Proactive Recovery and Monitoring the Flow of Medical reports, intended to avoid that the users forget to provide important information to the PHR system.
   Usually the type of user is located within the groups that hopefully will provide information. Basically these groups are:
   - Users without relevant pathologies
   - Children
   - Adolescents
   - Women in childbearing age.
   - Users with slight chronic pathologies
   - Users with moderate chronic pathologies
   - Users with acute chronic pathologies
   - Users over 65 years of age
   And obviously any possible combination among the above groups.
   Each one of these groups have an expected casuistic and a probability of a certain cadence as far as the entry of information is concerned (from totally unexpected diseases, at random or accidents, to data which probability of arrival is 100% such as vaccinations for example).
   The Center for Administration Control, following the pre-established guidelines and protocols gets in contact with the doctor who in turn gets in touch with the "doctor's pool" existing in the system. Regular phone calls are made (every three months as a minimum) to establish three essential courses of actions within the follow-up process:
   - To determine whether there has been any episode that has not been introduced.
   - To help the user to introduce it if that has been the case.
   - To reinforce the information concerning the PREVENTION and increase the quality of the assistance provided.
7.3. A functionality for the Classification of Types of Reports and ICD Codification that allows the user and the health professional, at once and at a glance to take a look to important volume of clinical information that helps to get a quick and clear idea of type of patient involved. This information created by the subsystem "Graphics of Types of Report" generates a "snapshot" or picture of the user's medical profile of important value at the time of considering factors of risks or important personal backgrounds which may be of great value in the patient's life in cases of new medical episodes.
   Basically, the classification makes a differentiation between the following fields:
   - Acronym of 3 letters: that defines the type of report (LAB = Laboratory, X Rays = X Rays Radiology, MRI = Magnetic Resonance Imaging).
   - Exact date
   - Grouping of episodes
   It is important to point out that the system does not only provides quick and organized information over the main medical specialties that affect the user and the distribution within the time, but also it gathers the events in episodes of higher level (for example admissions)
   The doctors of the system's "pool" on daily basis review the reports recorded in the database and add the information concerning the protocols described above. In those cases where there are doubts about whether or not several isolated events represent episodes that may be associated the doctors get in contact with the user (by telephone, e-mail etc.,) to help him/her to round up the information.
7.4. A Call Center functionality of medical to support in case of incidents by introducing or deleting information.
   When a call from the user is received, the operator of the Call Center decides if the problem is of administrative nature, if dealing with a technical problem or if needs medical care.
   The incidents of medical nature are those dealing with the administration of the medical data when interacting with the PHR, either information from the Patient's Diary or problems in connection with the introduction of clinical reports into the account of the user.
   Once the administrative operator has determined that the problems is of health nature the call is transferred to the Administration Doctor that in turn decides whether or not it will be necessary to get the user in contact with any of the Operators of the Health Data (that do not necessarily belong to the "pool of doctors" of the system) to help the user to resolve the incident.
7.5. - A functionality to monitor and manage the CAC system and resolve the incidents that may exist within the flow of information between the doctors and the patients of the CAC system (Continuity of Assisted Care). The base of this functionality is the quick and simple creation of protocols for the personal monitoring of diseases consisting of a private relation between the doctor and the patient which is simplified through the Platform of the CAC system that is not in any case considered as a medical act.
   The services associated with the CAC system provide support, supervise and control the correct flow of information that is created once the follow-up and control relation has been established between the doctor and the patient.
   The performance sequence is the following:
   - Regular review of all those protocols or templates that have been activated in a specific period of time.
   - Introduction into the system of the information required to monitor the periodicity recommended by the doctor.
   - Automatic review of the compliance by the part of the patient of the periodicity recommended.
   - In those cases where a lack of reception of data is detected the Call Center gets in touch with the user as a first phase.
   - If the patient decides to abandon the follow-up procedure, the doctor is informed accordingly and the protocol is cancelled.
   In case of existence of any error or problem of logistic nature, the user is provided with support and the appropriate assistance is offered so the user may give the
   information required. Additionally, a report about the involvement of the control system is sent to the doctor to facilitate the data flow, informing him that is not a case of desertion or rejection by the part of the patient. In the latter case the patient is reclassified to ensure that he/she is more closely monitored in the future.
   For the purposes of offering more exact details of the description a reference is made afterwards to the sheets or drawings that include although are not limited to:

### IN THE DRAWINGS:

Figure 1 is a chart that represents the functional operation consisting of the introduction of data via Safe Fax.
Figure 2 is a chart that represents the functional operation consisting of the introduction of data through CONNECTIVITY
Figure 3 is a chart representing the functional operation consisting of the prevention by profile.
Figure 4 is a chart representing the functional operation 1 of the Continuity of Assisted Care (CAC). Part 1
Figure 5 is a chart representing the functional operation 2 of the Continuity of Assisted Care (CAC) Part 2.
Figure 6 is a chart that represents the functional operation dealing with the systems integration
Figure 7 is a chart that represents the functional operation dealing with Adjustment, Embedding and Mimicry
Figure 8 is a chart that represents the professional functional operation.

### PREFERENTIAL REALIZATION OF THE INVENTION

Hereunder we explain a preferential realization of the invention in accordance with a summarized and schematic version consisting of:
- A system for the introduction of data via Safe Fax (Fig 1) that includes the user (1); the module of the medical history or the PHR itself (2); a Safe Fax (3) (fix number plus identifier exclusively for user), other reports of the user coming from several origins (4); plus the central database (5).
- A system for the introduction of data via Connectivity (Fig 2) that includes: the user (1); a standard connection component for acceptance of data tabulated and structured (2); a Safe Fax (3) (fix number and identifier exclusively for the user), other reports of the user (1) coming from several origins (4); central database (5);information system of the hospital (HIS) plus a connection and extraction element for data.
- A prevention system per profiles (Fig 3) that includes: the user (I); group of data defining the type of user according to a pathology (2); a proprietary system for selection of profiles, tabulated in agreement with the data content (3); a process that assigns a prevention guide associated to a profile (4); the central database (5); process data base and instructions over prevention (6); selection of a package of instructions over prevention (7); and delivery of a prevention package appropriate for a specific user (8) (via web, SMS, etc.)

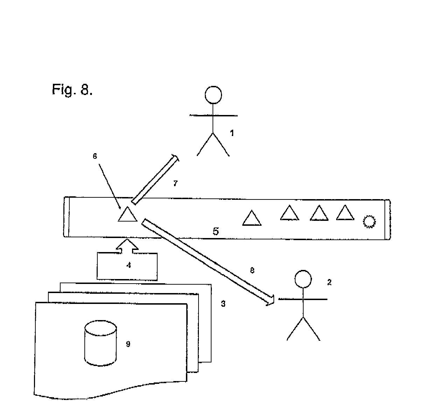

System for Continuity of Assisted Care (CAC) Part 1 Fig 4, that includes the user (1), the doctor (2); proprietary system for the creation of a form that gathers the patient's data (3); a process for the conversion of the form into a simple questionnaire of questions and answers (4); a central database (5); a questionnaire assigned to the patient based on the data provided (6); and delivery of the questionnaire to the user (1) (via web, SMS, etc.) (7).
- A system for the Continuity of Assisted Care (CAC) Part 1 Fig. 5 that includes the user (I); the doctor (2); the registration of personal data of the form in the database (3); a process for the conversion of personal data into the clinic guidelines to be followed up (4); a central data base (5); aggregation of all clinical parameters of the users (1) to a clinical report for follow-up (6); delivery of follow-up clinical report to the doctor (2) (via web, SMS, etc.) (7).
- A system for the aggregation of processes (Fig 6) that includes: the user 81); a standard connector for the acceptance of tabulated and structured data (2); safe fax (3); (fix number plus identifier exclusively for the user); other reports of the user coming from different origins (4); central database (5); system of hospital information (HIS) (6); system hospital information (different to the above) (6b); process for the connection and specific extraction f tabulated data (7) and process for data homologation, normalization and information (8).
- System for adjustment, embedding and Mimicry Fig 7 that includes the native web system (1); a different destination or hosting web system (2); thematic block 3 (5); thematic block n (6); information system, alarm and reminder 1 (7); information system, alarm and reminder n (8) and a process for assigning and relocating the information blocks from the system of origin to the hosting system following a protocol and an established directive.
- Professional system Fig 8 that includes: the user (1), the doctor (2); a system to handle the clinic information (based on the resident web) (3); a process for the generation of standard reports based on data registered by the doctor (4); basing of central data (5); report coming from the system for management of the doctor's clinic information (6); availability of report to user (7); availability of report to the doctor (8) and data base of doctor (9).

After conveniently describing the nature of the invention it is pointed out for the relevant purposes that such invention is not restricted to the exact details given throughout this account, but very much on the contrary, as the modifications deemed appropriate will be introduced provided they do not change the essential characteristics of the invention, characteristics that are claimed hereunder.

## Claims

1. SYSTEM FOR THE FULL CONTROL AND MANAGEMENT OF MEDICAL RECORDS OF PATIENTS IN HEALTHCARE CENTERS, HOSPITALS, OUTPATIENTS CENTERS AND THE GENERAL HEALTHCARE SYSTEM and more specifically a computer program that includes the following CHARACTERISTICS:
1. System that comprises the introduction of data
1.1. through the web
1.2. through a Safe Fax
1.3.through connectivity
2. System that includes the following operations:
2.0 proprietary of the medical history and extraction of information from the web.
2.1. full report
2.2. specific extractions
2.3. interface describing the report
3.0 System that includes the following operations of the Health Diary:
3.1 summary screen
3.2. main screen
4 System that includes the following prevention functionalities:
4.1 general
4.2 per profile
5. System that includes the following functionalities:
5.1. for extraction of information from emergencies through a PIN number and
5.2 for continuity of assisted care (CAC) (1), (2), (3) and (4)
6. System that includes the following functionalities:
6.1. incorporation of processes
6.2 adjustment, embedding and mimicry
6.3. professional (1), (2) and (3)
6.7. from the web and CAC to recommend protocols
6.8. from the hosting and connectivity webs
6.9 for technical maintenance and technological support
6.10 for monitoring and managing the correct connectivity for data coming from health information systems.
7. System that includes the following Functionalities:
7.1. telephone recommendations depending on patient's medical history
7.2. for proactive recovery and monitoring of flow of medical reports.
7.3 for classification of types of reports and ICD codification
7.4. "Call center" to obtain medical support in cases of incidents with introduction and extraction of information.
7.5. monitoring and management of the CAC's systems and resolution of incidents in the flow of information between doctors and patients.

2. SYSTEM FOR THE FULL CONTROL AND MANAGEMENT OF MEDICAL RECORDS OF PATIENTS IN HEALTHCARE CENTERS, HOSPITALS, OUTPATIENTS CENTERS AND THE GENERAL HEALTHCARE SYSTEM, in accordance with Claim 1, consisting of the introduction of data via the Web which main CHARACTERISTIC is that it allows access to a form to choose a file to be added to its particular database in the following formats:
JPEG
GIF
TIFF
BMP
PDF
That additionally allows the introduction of certain information associated to the document unloaded in the server.
Date of report
Doctor author
Specialty
Center of origin
Type of report
All the above information will be classified within the database of the system as information that has not been verified.

3. SYSTEM FOR THE FULL CONTROL AND MANAGEMENT OF MEDICAL RECORDS OF PATIENTS IN HEALTHCARE CENTERS, HOSPITALS, OUTPATIENTS CENTERS AND THE GENERAL HEALTHCARE SYSTEM, in accordance with Claim 1 consisting of the introduction of data via a safe fax which main CHARACTERISTIC is that the user has a single code of identification as well as an exclusive and private fax number made up of 9 digits of the heading plus an individual code that is specially assigned to him.

4. SYSTEM FOR THE FULL CONTROL AND MANAGEMENT OF MEDICAL RECORDS OF PATIENTS IN HEALTHCARE CENTERS, HOSPITALS, OUTPATIENTS CENTERS AND THE GENERAL HEALTHCARE SYSTEM, in accordance with Claim 1 consisting of the introduction of data via connectivity which main CHARACTERISTIC is that it has connectivity links or direct connectivity with the system and that it deposits a copy of the report directly in the database of the central system.

5. SYSTEM FOR THE FULL CONTROL AND MANAGEMENT OF MEDICAL RECORDS OF PATIENTS IN HEALTHCARE CENTERS, HOSPITALS, OUTPATIENTS CENTERS AND THE GENERAL HEALTHCARE SYSTEM in accordance with Claim 1 consisting of a verification of the owner of the medical report which main CHARACTERISTIC is that it allows to have a quick and precise image of its medical profile by only looking at a diagram, that in one axle shows a time scale and on the other the columns that depict the several medical practices that represent the areas in which the specialties are divided that in turn comprise the following elements:
- Acronym of 3 letters: that defines the type of report (LAB, RAX= Radiology X Rays, RMN = Magnetic Resonance Imaging (MRI)
- Exact date, and Additionally, in case of surgery the following reports:
From urgencies
From the laboratory
Preanesthetic preparation
From Surgery
Hospitalization continuity
Discharge

6. SYSTEM FOR THE FULL CONTROL AND MANAGEMENT OF MEDICAL RECORDS OF PATIENTS IN HEALTHCARE CENTERS, HOSPITALS, OUTPATIENTS CENTERS AND THE GENERAL HEALTHCARE SYSTEM, according to Claim 1, consisting of the extraction of information through the web - via specific extraction and via descriptive interface of the full report which main CHARACTERISTIC is that offers two options:
A: extraction of the full report
B: specific extraction and search of reports
With an interface that in both cases allows to choose:
- Range of dates of insertion for searches (initiation / final)
- Range of dates of creation for searches (initiation / final)
- Specialties
- Center of origin
- Type of report
- Doctor / Inspector of report
- CIE Code
Also allowing:
- Codified information
- Text of report
- Images or documents attached

7. SYSTEM FOR THE FULL CONTROL AND MANAGEMENT OF MEDICAL RECORDS OF PATIENTS IN HEALTHCARE CENTERS, HOSPITALS, OUTPATIENTS CENTERS AND THE GENERAL HEALTHCARE SYSTEM, according to Claim 1, consisting of a verification of the Diary of Health - summary screen that includes a double option- which main CHARACTERISES is that is has:
- A summary screen
that allows, from the central page that gathers the global information, to access summaries of personal health which in turn are broken down into specific windows for:
- Control of weight
- Control of healthy life habits
- Control of diet
- Control of physical exercise
- Personal agenda that allows to enter appointments and reminders on specific dates and associate them with alarms and,
- Main screen that allows an access to more precise information and to follow up tables of each one of the basic parameters, by using a form where the data may be updated on regular basis.

8. SYSTEM FOR THE FULL CONTROL AND MANAGEMENT OF MEDICAL RECORDS OF PATIENTS IN HEALTHCARE CENTERS, HOSPITALS, OUTPATIENTS CENTERS AND THE GENERAL HEALTHCARE SYSTEM, in accordance with Claim 1 that includes a functionality for general prevention which main CHARACTERISTIC is that it includes processes that provide information about any protocol of prevention published, which information is not sorted but it arrives exactly as presented by the origin or source; updating and renewing the information by seasonal modifications shown in two areas:
- Window of summary on the personalized page of the user
- Window of detailed explanations

9. SYSTEM FOR THE FULL CONTROL AND MANAGEMENT OF MEDICAL RECORDS OF PATIENTS IN HEALTHCARE CENTERS, HOSPITALS, OUTPATIENTS CENTERS AND THE GENERAL HEALTHCARE SYSTEM, in accordance with Claim 1, that comprises a prevention functionality per profile consisting of processes that inform about any protocol of prevention published and which main CHARACTERISTIC is that in this case the information is sorted out, crossed and compared with the information stored in the system that basically corresponds to:
- Age
- Sex
- Weight
- Smoking habits
- Pharmacological habits
- Stress
- Diet habits
- Personal background
- Family background
The additional prevention is made with a high degree of sophistication and specialty. The functionality at the same time includes:
- A summary screen with the results of the information crossed with the database of all potential protocols of prevention for any group of risk
- A specific screen explaining each protocol in particular

10. SYSTEM FOR THE FULL CONTROL AND MANAGEMENT OF MEDICAL RECORDS OF PATIENTS IN HEALTHCARE CENTERS, HOSPITALS, OUTPATIENTS CENTERS AND THE GENERAL HEALTHCARE SYSTEM, in accordance with Claim 1 that includes a functionality consisting of the extraction of information from emergencies through a PIN code which main CHARACTERISTIC is that it offers the users, after registration, besides the name and the password, a special number called PIN of emergency that allows the immediate remittance of the full medical report stored in the system to the address provided. Once used the system disables this Emergency PIN number and generates a new one.

11. SYSTEM FOR THE FULL CONTROL AND MANAGEMENT OF MEDICAL RECORDS OF PATIENTS IN HEALTHCARE CENTERS, HOSPITALS, OUTPATIENTS CENTERS AND THE GENERAL HEALTHCARE SYSTEM, in accordance with Claim 1 that has a functionality that consists of a verifying the Continuity of Assisted Care (CAC) in three equal phases where the proprietary process of the CAA is **CHARACTERIZED by** having two main components:
A) a process of individualized pattern through the Web that allows to establish a simple and personalized protocol that includes the following fields:
A) Name of protocol
B) Name of parameter
C) Maximum value to be assessed
D) Minimum value to be assessed
E) Follow up mode
F) Regular follow up option
B) Review mode of the patient's data in groups and in detail, besides: Access may be obtained to a web process that allows:
A) To review a global chart of the health condition, the progression and the remarks made with reference to the patients under treatment
B) To review in detail the partial progression of a particular patient

12. SYSTEM FOR THE FULL CONTROL AND MANAGEMENT OF MEDICAL RECORDS OF PATIENTS IN HEALTHCARE CENTERS, HOSPITALS, OUTPATIENTS CENTERS AND THE GENERAL HEALTHCARE SYSTEM, according to Claim 1 that includes a functionality consisting of the integration of processes **CHARACTERIZED by** containing the elements required for the incorporation of the information with any other method used in hospitals, systems of first aid, or systems that handle clinical histories in clinics or doctor's offices, which includes an exact definition of the fields to be imported:
- Name of the report
- Name of the patient
- Basic details of the patient's affiliation
- Text of the report to be delivered
- Set of the files attached
- ICD code
- Internal code for classification and indexing
- Specialty
- Date
- Type
- Index of opening and closing of episode
Additionally it includes an APIS (Application Programming Interfaces) process that allows the rest of the processes to communicate in a fluent manner with the system that in turn offers a full setting for the communication of the fields described as well as a general definition for future developments.

13. SYSTEM FOR THE FULL CONTROL AND MANAGEMENT OF MEDICAL RECORDS OF PATIENTS IN HEALTHCARE CENTERS, HOSPITALS, OUTPATIENTS CENTERS AND THE GENERAL HEALTHCARE SYSTEM, according to Claim 1 that includes a functionality of adjustment, embedding and mimicry **CHARACTERIZED by** a development in exchangeable blocks of codes and XML templates for a simple incorporation in the Web portals of other institutions, thus allowing quick modifications of:
- The color schemes
- The issues schemes
- The size of letters and the type of headings
- The schemes of relational placement
The functionality may be used in a different area although using the same "motor" of operation and the same database.

14. SYSTEM FOR THE FULL CONTROL AND MANAGEMENT OF MEDICAL RECORDS OF PATIENTS IN HEALTHCARE CENTERS, HOSPITALS, OUTPATIENTS CENTERS AND THE GENERAL HEALTHCARE SYSTEM, according to Claim 1, that includes a global professional functionality **CHARACTERIZED by** an application made up of charts or frames as well as menus. The data provided includes:
- General information
- Name of the application and of the institution the system is connected to
- Type of profile of user
- Menus of the application
- There are three menus:
File menu that offers the options of saving, changing the general data, changing the password, the data base and an exit.
The submenu allows to record any change
The submenu that changes the general data allows us to modify the general information of the user; the date concerning the first name, the family name, the association number and the electronic mail may also be modified.
The submenu that changes the password allows us to modify our code of access to the application.
The submenu for the medications data takes the user to the page of publications from where access may be obtained to the medications and their active ingredients The Exit submenu is used to get out of the application The Tools Menu has a submenu for preferences and editing the health institution:
The Preferences submenu allows access to organize the characteristics of the specific application:
- Location of the database of medications or access to a free version
- The configuration concerning the remittance of data allows us to organize the data or the parameters of the server of the electronic mail and the account Language: the functionality allows us to choose the language of the texts and the messages of the application.
The submenu to Edit Health Institution allows us the access to modify the clinic's data, the name, the description and to add or delete the insurance companies the clinic works with.
The Help menu allows us to see the technical information of the application.

15. SYSTEM FOR THE FULL CONTROL AND MANAGEMENT OF MEDICAL RECORDS OF PATIENTS IN HEALTHCARE CENTERS, HOSPITALS, OUTPATIENTS CENTERS AND THE GENERAL HEALTHCARE SYSTEM, in accordance with Claim 1, that includes a web functionality of the CAC (Continuity of Assisted Care) to recommend individualized protocols through the web CHARACTRIZED by using a simple protocol which is accessible through the web that allows to choose a specific patient and set up a simple personalized protocol that includes the following fields:
A) Name of protocol
B) Name of parameter
C) Maximum value to be assessed
D) Minimum value to be assessed
E) Follow- up mode
F) Regular follow-up option

16. SYSTEM FOR THE FULL CONTROL AND MANAGEMENT OF MEDICAL RECORDS OF PATIENTS IN HEALTHCARE CENTERS, HOSPITALS, OUTPATIENTS CENTERS AND THE GENERAL HEALTHCARE SYSTEM, in accordance with Claim 1, that includes a functionality for monitoring and managing the correct connectivity of the information coming from other information systems **CHARACTERIZED by** having a specific service that establishes a "monitoring channel" or data flow for verification with any data supplier, this is, with any health information system with direct incorporation of its clinic data within the process; additionally an "independent monitoring channel" is set up for data coming from the professional proprietary process. The data verified on permanent basis is:
- Status of the "open" operation of the channel
- No intrusion filter
- Control of the expected correct volume of information in a period of time
- Random control of blocks of patients in an anonymous manner.
An analysis report is issued on regular basis indicating the magnitudes picked up during the continued verification process as well as the performance of the connection channels.

17. SYSTEM FOR THE FULL CONTROL AND MANAGEMENT OF MEDICAL RECORDS OF PATIENTS IN HEALTHCARE CENTERS, HOSPITALS, OUTPATIENTS CENTERS AND THE GENERAL HEALTHCARE SYSTEM, in accordance with Claim 1, that includes a functionality for telephone recommendations according to the patient's medical history CHARACETERIZED by allowing a quick deployment and star-up of the user's PHR through a specific protocol by apparatus, putting in place the best monitoring procedure to be used in the continuity that initially has determined the typology of the user, which basically is the following:
- Users without relevant pathologies
- Children
- Adolescents
- Women in childbearing age.
- Users with slight chronic pathologies
- Users with moderate chronic pathologies
- Users with acute chronic pathologies
- Users over 65 years of age
And obviously all possible combinations among the groups and applying the previously established guidelines and protocols:
- To know if there has been any episode that has not been introduced
- To help the user to introduce it if that has been the case
- To reinforce the information concerning prevention increasing the quality of the assistance provided.

18. SYSTEM FOR THE FULL CONTROL AND MANAGEMENT OF MEDICAL RECORDS OF PATIENTS IN HEALTHCARE CENTERS, HOSPITALS, OUTPATIENTS CENTERS AND THE GENERAL HEALTHCARE SYSTEM, in accordance with Claim 1, that includes a functionality for the classification of the types of reports and an ICD codification **CHARACTERIZED by** allowing the user as well as the professional to have access and take a look at an important volume of clinical information. The information is basically divided in the following fields:
- Acronym of 3 letters: that defines the type of report (LAB, RAX= Radiology X Rays, RMN = Magnetic Resonance Imaging (MRI)
- Exact date
- Groups of episodes
